# EUROPEAN PATENT APPLICATION

(11) **EP 1 111 069 A1**
(43) Date of publication of application: **27.06.2001**
(21) Application number: 99125672.8
(22) Date of filing: 22.12.1999
(51) Int. Cl.: C12Q 1/68

(54) **Modified nucleic acids and their use**

(71) Applicant: BioChip Technologies GmbH, 79108 Freiburg i. Br. (DE)
(72) Inventor: KLAPPROTH, Holger, 79108 Freiburg (DE)
(74) Representative: Stürken, Joachim

(57) **Abstract**

The invention relates to a process for the modification of double-stranded nucleic acids with groups that allow a covalent binding of these nucleic acids to surfaces on supports or other substrates. In a further embodiment, the invention relates to a modified nucleic acid obtainable according to the method or process just mentioned. In a further embodiment, the present invention relates to a support carrying a covalently bonded modified nucleic acid, especially a DNA or RNA, where the nucleic acid is bonded via one or more modified nucleosides to the surface of said support In a further embodiment, the invention relates to a method of manufacturing a support carrying a covalently bonded modified nucleic acid, especially a DNA or RNA, where the nucleic acid is bonded via one or more modified nudeosides to the surface of said support. Furthermore, the invention relates to a matrix that may be gel-like and that is obtainable by cross-linking of a modified nucleid acid according to the invention with a cross-linker. The invention also relates to a method of manufacture of such a matrix. The invention also relates to a kit employing a support or matrix according to the invention, especially for detection or purification of nucleic acids, or nucleic acid binding (e.g. regulatory) proteins. In another embodiment, the present invention relates to a kit for introducing modified nucleosides into a nucleic acid and then covalently binding such a nucleic acid to a support.

## Description

The invention relates to a process for the modification of a nucleic acid with groups that allow a covalent binding of these nucleic acids to surfaces on supports or other substrates. In a further embodiment, the invention relates to a modified nucleic acid obtainable according to the method or process just mentioned. In a further embodiment, the present invention relates to a support carrying a covalently bonded modified nucleic acid, especially a DNA or RNA, where the nucleic acid is bonded via one or more modified nucleosides to the surface of said support In a further embodiment, the invention relates to a method of manufacturing a support carrying a covalently bonded modified nucleic acid, especially a DNA or RNA, where the nucleic acid is bonded via one or more modified nucleosides to the surface of said support. Furthermore, the invention relates to a matrix that may be gel-like and that is obtainable by cross-linking of a modified nucleid acid according to the invention with a cross-linker. The invention also relates to a method of manufacture of such a matrix. The invention also relates to a kit employing a support or matrix according to the invention, especially for detection or purification of nucleic acids, or nucleic acid binding (e.g. regulatory) proteins. In another embodiment, the present invention relates to a process and a kit for introducing modified nucleosides into a nucleic acid and then covalently binding such a nucleic acid to a support.

### Background of the invention:

Two fundamental processes are known for the coupling of nucleic acids to surfaces: covalent or non-covalent coupling (usually haptene-mediated).

The method of covalent coupling encompasses two different variants. Either the coupling group is bound directly to the DNA, or the DNA, after a chemical modification, is brought into some intermediate status that is capable of coupling by modification after DNA synthesis (customarily, here an acid or amino group is brought into the status of being capable of coupling by reaction with a cross-linker).

### Methods without cross-linkers:

1.) Synthesis of oligonucleotides which are furnished terminally with an amino linker during a process of manufacture. The coupling then takes place on amino-reactive surfaces (e.g. epoxy- or activated ester-functionalised surfaces).
2.) Synthesis of oligonucleotides which are furnished terminally with a thiol group during the process of manufacture (e.g. gold-plated surfaces or maleinimide-layered surfaces).

### Methods requiring cross-linkers:

3.) Synthesis of oligonucleotides which, during the process of manufacture, are furnished terminally with a phosphate group (instead of a hydroxy group). Subsequently, a derivation of the acid group is made, e.g. with EDC, so that an amino-reactive (or otherwise reactive) group is formed.
4.) Synthesis of oligonucleotides having a group as described under 1. and 2. and that are coupled to surfaces functionalised with nucleophiles (e.g. by way of a hetero-bifunctional cross-linker).
5.) Chemical modification of the sugar moiety yielding abasic (e.g. apurinic) positions in nucleic acid strands in order to introduce amino groups for coupling DNA via the modified sugar moieties to surfaces. This method also requires chemical cross-linkers for coupling the DNA to aldehyde functions. As by way of this method abasic sites are created in a DNA strand, the DNA at these sites is no longer appropriate for detection reactions based on hybridisation.

### Non-covalent coupling techniques for DNA

This process customarily is based on the binding of haptenes (e.g. biotin) to acceptor molecules (e.g. avidin) in order to furnish a covalent binding of nucleic acids to supports. These bonds are non-covalent and, as a result, much less stable than covalent bonds.

Non of the prior art processes is suitable for furnishing any nucleic acid strand with coupling groups capable of covalent binding without destroying the integrity of the nucleic acid strand. Methods based on the chemical synthesis of modified oligonucleotides are not appropriate for the coupling of larger DNA segments. Here especially the reactivity of amino groups of the distinct bases of the DNA is very annoying. The chemical (non-enzymatic) modification of nucleic acids requires abasic (e.g. apurinic) positions in the DNA. For that reason, the coupling properties of the DNA and the hybridisation properties are significantly disturbed. It follows that there is big interest and an unsatisfied need to modify DNA such that it is appropriate for covalent binding to surfaces or supports. A process for introducing randomized coupling groups into any desired nucleic acid strand without interfering with its integrity is, given the methods known hitherto which are based mainly on oligonucleotide synthesis, unknown.

It is therefore an unsolved problem to present a method that allows to modify DNA or RNA molecules by way of an enzymatic process in such a way that, distributed over the whole molecule, coupling groups are present that allow for the covalent binding to surfaces.

To provide the solution for this problem is the basic objective of the present invention.

Some further advantages of the method according to the invention are:
The method involves the smallest possible modification of nucleotides in order to allow for covalent chemistry with nucleic acids while still maintaining the integrity of the double helix.
The acceptance of especially AA-dUTP by DNA polymerases is very high, so that an efficient insertion is possible (this, however, often is not true for larger modifications).
It is possible to label DNA/RNA strands covalently without substantially changing the structure of any double helix.
Double-stranded nucleic acids can be bonded to the surface of a support homogeneously and specifically, for example in order to produce oligonucleotide arrays.

Some of the fields of application of the present invention are:
- covalent coupling of polynucleotides to surfaces of or on solid supports, e.g. in the manufacture of oligonucleotide arrays.
- covalent modification of nucleic acids with ligands that are not suited for enzymatic reaction or have impact on the efficiency of an enzymatic reaction (e.g. rhodamine). For example, if modified nucleoside trisphosphates are used in the preparation of a modifed nucleic acid mentioned below that carry both amino and thiol coupling groups, then it is first possible to modify the thiol group with a SH-specific marker, e.g. maleimido-rhodamine, and then couple the whole molecule to a support by reacting the remaining amino groups. On the other hand, it is also possible to simply modify the modified nucleotides by introducing markers, such as activated rhodamine, in order to get the corresponding labelled nucleic acid.
- crosslinking of polynucleotide strands for the manufacture of a matrix that may be gel-like in order e.g. to use said matrix for affinity chromatography.

These and further advantages and fields of application are described in more detail below:

### General decription of the invention

(A) The invention relates in particular to a method or process for the manufacture of a modified nucleic acid, especially a DNA or RNA, especially carrying a coupling group capable of binding covalently to a support or to other molecules, comprising the steps of multiplying (or translating) an available nucleic acid, especially a DNA or RNA, using a DNA or RNA polymerase, where, during the process, either one modified nucleoside trisphosphate or more than one non-identical modified nucleoside trisphosphates (that is, nucleoside trisphosphates of one or more types) are used as substrate in order to obtain a nucleic acid wherein at least one of the modified nucleosides is present and at least one modified nucleoside is bonded non-terminally.
   Preferably, any primers used for the synthesis are such that no modified nucleoside results at the end (terminally) of the respective nucleic acid strand.
   The main advantage of this method or manufacturing process is that, if also the corresponding unmodified nucleoside trisphosphates are present, the modified nucleoside or nucleosides are built in at random positions so that, taken over the whole of the resulting nucleic acids, there is a randomized influence on the properties of the respective nucleic acid, e.g. with regard to hybridisation. Depending on the molar relationship of modified to unmodified nucleoside trisphosphates, only a certain percentage of the nucleosides in the resulting nucleic acid at a given position is present in modified form.
   If one primer is used in the method, the resulting nucleic acid (especially a DNA or RNA) is modified only on one strand. If two primers are used, the resulting nucleic acid (especially a DNA) is modified on both complementary strands if present.
(B) In a further embodiment, the invention relates to a modified nucleic acid obtainable according to the method or process described under (A), especially a double-stranded DNA that comprises modified nucleosides of one or more types either in one or in both strands, or an RNA that carries such modified nucleosides, especially carrying a coupling group capable of binding covalently to a support or to other molecules, with the proviso, that at least one of the binding modified nucleosides is not terminally bound to the respective modified DNA or RNA, or preferably that no modified nucleoside is present terminally in the respective modified nucleic acid strand(s).
(C) In a further embodiment, the present invention relates to a support carrying a covalently bonded modified nucleic acid, especially a DNA or RNA, where the nucleic acid is bonded via one or more modified nucleosides (which may be of one or more types) to the surface of said support, with the proviso, that at least one of the binding modified nucleosides is not terminally bound to the respective modified DNA or RNA, or preferably that no modified nucleoside is present terminally in the respective modified nucleic acid strand(s).
(D) In a further embodiment, the invention relates to a method of manufacturing a support carrying a covalently bonded modified nucleic acid, especially a DNA or RNA, where the nucleic acid is bonded via one or more modified nucleosides (of one or more types) to the surface of said support, with the proviso that at least one of the binding modified nucleosides is not terminally bound to the respective modified DNA or RNA, or preferably that no modified nucleoside is present terminally in the respective modified nucleic acid strand, comprising the step of reacting said modified nucleic acid with a support.
   In the case where the modified nucleic acid carries modified nucleosides only in one strand, the resulting support with the covalently bonded modified nucleic acid is especially suited for hybridisation reactions, allowing for low hybridisation temperatures to be chosen and thus shorter duration of analysis, making such modified supports especially appropriate for High Throughput Screening (HTS).
   For this purpose, the modified nucleic acid is melted before the coupling reaction to the support takes place, so that only the single strand(s) react with the support.
(E) Furthermore, the invention relates to a matrix (that may preferably be gel-like) that is obtainable by cross-linking of a modified nucleid acid (preferably one wherein at least one of the binding modified nucleosides (which may be of one or more types) is not terminally bonded to the respective modified DNA or RNA, or more preferably where no modified nucleoside is present terminally in the respective modified nucleic acid strand) according to the invention with a cross-linker. Preferably, the cross-linking takes place between more than one modified single or double strand DNA molecules or modified RNA molecules.
   An advantage of such a matrix is that it has very high adsorption capacity. Such gels can e.g. be fixed on supports, such as chips or membranes (e.g. modifed cellulose).
(F) The invention also relates to a method of manufacture of such a matrix, comprising the steps of reacting a nucleic acid incorporating at least one modified nucleoside as defined herein with the proviso that at least one modified nucleoside is not terminally bound at the respective nucleic acid, with a hetero- or especially homo-bi-functional cross-linker.
(G) The invention also relates to a kit comprising a support (embodiment (C)) or matrix (embodiment (E)) according to the invention, especially for detection or purification of nucleic acids, and the use thereof for analysis or purification purposes, e.g. analysis or purification of nucleic acid or protein samples.
(H) In another embodiment, the present invention relates to a kit for introducing modified nucleosides into a nucleic acid and, if desired, then covalently binding such a nucleic acid to a support.
(I) In still another embodiment, the present invention relates to a combination of embodiment (A) and ambodiment (B) above, that is, to a process for the manufacture of a modified nucleic acid, especially a DNA or RNA, especially carrying a coupling group capable of binding covalently to a support or to other molecules, comprising the steps of multiplying (or translating) an available nucleic acid, especially a DNA or RNA, using a DNA or RNA polymerase, where, during the process, either one modified nucleoside trisphosphate or more than one non-identical modified nucleoside trisphosphates are used as substrate in order to obtain a nucleic acid wherein at least one of the modified nucleosides is present and at least one modified nucleoside is bound non-terminally; and then manufacturing a support carrying a covalently bonded modified nucleic acid, especially a DNA or RNA, where the nucleic acid is bonded via one or more modified nucleosides (of one or more types) to the surface of said support, with the proviso that at least one of the binding modified nucleosides is not terminally bound to the respective modified DNA or RNA, or preferably that no modified nucleoside is present terminally in the respective modified nucleic acid strand, comprising the step of reacting said modified nucleic acid with a support.

In all these embodiments, it is a prefeable result that the integrity of the nucleic acid, if compared to the natural or synthetic un-modified nucleic acid, is essentially maintained.

### Detailed description of the invention

The following definitions provide the preferred embodiments of the expressions and symbols used above; in any preferred embodiments of the invention, as well as in the claims, it is possible to use one or more of these preferred definitions in order to come to preferred embodiments of the invention.

Whereever any compounds are mentioned herein, these compounds may also be present as their salts where salt-forming groups are present, which salts are also part of the present disclosure.

In accordance with the present invention, the term "nucleic acid" comprises any feasible derivative of a nucleic acid to which a nucleic acid probe may hybridize. In addition, the nucleic acid may be any derivative of a nucleic acid capable of binding to said nucleic acid.

It is preferred that the nucleic acid used according to the method of the invention is a DNA or RNA. Examples of RNA are rRNA or mRNA, Examples of DNA are cDNA, genomic DNA, antisense DNA and the like. Especially preferred are either DNA or RNA.

Where the term "modified nucleic acid" is mentioned, a nucleic acid obtainable by the process according to embodiment (A) above or its preferred embodiments is meant, that is, a nucleic acid carrying modified nucleosides that result from use of the corresponding nucleoside trisphosphates in the polymerase reaction. If covalently bonded to a support, the term also encompasses the bonded nucleic acid obtaniable after reaction of such support with the free modified nucleic acid accoding to embodiment (D) above or its preferred embodiments.

By the term "integrity", it is especially meant that the sugar backbone is unchanged and the distances between the bases are practically unchanged when compared with unmodified nucleic acids, so that especially in the case of double strand or bubble formation the binding energy is changed by not more than 1 % when compared with the native (unmodified) nucleic acids.

The modified nucleoside trisphosphates useful for the embodiments of the present invention, especially for the process of manufacturing a modified nucleic acid in accordance with the present invention, contain one (or, in a broader aspect of the invention, more than one) coupling group(s) (appropriate for a reaction leading to covalent binding) that are bound via a spacer to the base parts of the molecule. Modified ribonucleoside trisphosphates and deoxyribonucleoside trisphosphates are preferred.

The spacers carrying the coupling groups should not be placed on ring positions that sterically, or otherwise, interfere with the normal Watson - Crick hydrogen bonding potential of the bases. Otherwise, the substituents will yield compounds that are inactive as polymerase substrates. Substitution at ring positions that alter the normal "anti" nucleoside conformation also must be avoided since such conformational changes usually render nucleotide derivatives unacceptable as polymerase substrates. Normally, such considerations limit substitution positions to the 5-position of a pyrimidine and the 7-position of a purine or a 7-deazapurine.

Preferably, a modified nucleoside trisphosphate has the following formula (I), 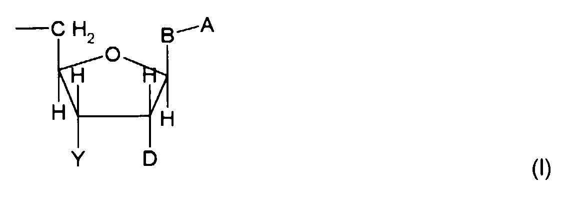 wherein
- Y: is ―O-P[(=O)(OH]-O-P[(=O)(OH]-O-P[(=O)(OH]-OH;
- A: is a spacer carrying a coupling group;
- B: is a base; and
- D: is hydroxy or hydrogen.

A, the spacer carrying a coupling group, should not be placed on ring positions that sterically, or otherwise, interfere with the normal Watson - Crick hydrogen bonding potential of the bases. Otherwise, the substituents will yield compounds that are inactive as polymerase substrates. Substitution at ring positions that alter the normal "anti" nucleoside conformation also must be avoided since such conformational changes usually render nucleotide derivatives unacceptable as polymerase substrates. Normally, such considerations limit substitution positions to the 5-position of a pyrimidine and the 7-position of a purine or a 7-deazapurine.

As bases B, purines, pyrimidines and 7-deazapurines are in principle useful, but pyrimidines and 7-deazapurines are preferred since purine substitution at the 8-position tends to render the nucleotides ineffective as polymerase substrates. Thus, although modified purines are useful in certain respects, they are not as generally useful as pyrimidines and 7-deazapurines. Moreover, pyrimidines and 7-deazapurines useful in this invention preferably must not be naturally substituted at the 5- or 7- positions, respectively. As a result, certain bases such as thymine, 5-methylcytosine, and 5-hydroxymethylcytosine are less useful.

Presently preferred bases are cytosine, uracil, deazaadenine and deazaguanine, especially uracil. A may include any of the well known bonds including carbon-carbon single bonds, carbon-carbon double bonds, carbon-nitrogen single bonds, carbon-sulfur single bonds, carbamide bonds, or carbon-oxygen single bonds. However, it is generally preferred that A include an olefinic bond at the α-position relative to B. The presence of such an α-olefinic bond serves to hold the coupling group away from the base when the base is paired with another in the well known double-helix configuration.

The coupling group consists of a group that is stable against hydrolysis in aqueous solution and that can couple covalently (i.e., leading to covalent bonds with the substrate), such as an amino or thiol group. Further possible groups are carboxy groups, aldehyde groups, hydroxy groups, phosphate groups, sulfonate groups or thioester groups, or nucleophilic groups (halo, e.g. bromo, chloro or iodo, or sulfonates, e.g. tosylate, or the like), or also azide or diene groups (appropriate for Diels-Alder couplings). Also possible are groups that are stable in aqueous solution and that by means of subsequent cleavage or activation become active groups, e.g. amino groups or protected by protecting groups that are known in the art, or disulfide groups that can subsequently be reduced to yield free mercapto groups.

Protecting groups, their introduction and their removal are, for example, described in standard textbooks, such as "Protective Groups in Organic Chemistry", Plenum Press, London, New York 1973, and in "Methoden der organischen Chemie", Houben-Weyl, 4th edition, Vol. 15/1, Georg-Thieme-Verlag, Stuttgart 1974 and in Theodora W. Greene, "Protective Groups in Organic Synthesis", John Wiley & Sons, New York 1981. A characteristic of protecting groups is that they can be readily removed, that is to say, without undesired secondary reactions taking place, for example by solvolysis, reduction, photolysis or also under physiological conditions, and that they are not present in the final products.

Especially, a protected amino group is protected by an amino protecting group, for example in the form of an acylamino, arylmethylamino, etherified mercaptoamino, 2-acyl-lower-alk-1-enylamino or silylamino group, or as an azido group.

It is preferred that the coupling groups are derived from or are a primary amino group, especially comprising the structure -CH₂-NH₂, since bonds with these groups are easily formed utilizing any of the well-known amine modification reactions.

Although these coupling groups are preferred, others can be used, including particularly other modifiable functionalities such as thiol, carboxylic acid, or epoxide functionalities, or azido groups appropriate for Diels-Alder coupling with diene groups, or diene groups appropriate for Diels-Alder coupling with azido groups.

The spacers carrying the coupling groups are preferably attached at specific positions, namely, the 5-position of a pyrimidine, the 8-position of a purine, or the 7-position of a deazapurine. As indicated previously, substitution at the position of a purine does not produce a modified nucleotide which is useful in all the methods discussed herein. It may be that the 7-position of a purine, which is occupied by a nitrogen atom, could be the point of linkage attachment. However, the chemical substitution methods employed to date are not suitable for this purpose. Spacers may have different lengts; preferably, they have a chain length of 3 or more atoms. Spacers are preferably unsaturated, partially saturated or saturated aliphatic groups. A spacer may also be a mono-, oligo- or a polymer made of an aliphatic monomer conjugated with an ester, ether of other bond, e.g. a polyether. One or more further C-atoms within the spacer may be replaced by a heteroatom, e.g by N, S or O.

The following spacers are preferred:

-NH-(CH₂)ₙ-X-(CH₂)ₚ-

-NH-(CH₂)ₙ-X-(CH₂)ₚ-NH-

-O-(CH₂)ₙ-X-(CH₂)ₚ-

-O-(CH₂)ₙ-X-(CH₂)ₚ-NH-

-S-(CH₂)ₙ-X-(CH₂)ₚ-

-S-(CH₂)ₙ-X-(CH₂)ₚ-NH-

-NHCO-(CH₂)ₙ-X-(CH₂)ₚ-

-NHCO-(CH₂)ₙ-X-(CH₂)ₚ-NH-

-NHSO₂-(CH₂)ₙ-X-(CH₂)ₚ-

-NHSO₂-(CH₂)ₙ-X-(CH₂)ₚ-NH-

-(CH₂)ₙ-X-(CH₂)ₚ-

-(CH₂)ₙ-X-(CH₂)ₚ-NH-

-CO-(CH₂)ₙ-X-(CH₂)ₚ-

-CO-(CH₂)ₙ-X-(CH₂)ₚ-NH

wherein n is 1 to 6, p is 0 or 1 to 6, and
X is CH₂, O, S, NH, CO, -CH=CH-, -(CH₂)ᵣ-, -(CH₂)ᵣ-NH-, -C(=CH-(CH₂)ᵣ)-, -C(=CH-(CH₂)ᵣNH)-, -N((CH₂)ᵣ)-, -N((CH₂)ᵣNH)-, -CH(-O(CH₂)ᵣ)ₛ)-, -CH(-O(CH₂)ᵣ) ₛ-NH)-, -CH(-S(CH₂)ᵣ) ₛ)- or -CH(-S(CH₂)ᵣ)-NH)-, wherein each of r and s, independently of the other, is a number from 1 to 20, preferably 1 to 6.

It is even more preferred that A be derived from a primary amine, and comprise the structure -CH₂-NH-, since such linkages are easily formed utilizing any of the well known amine modification reactions. Examples of preferred groups A are derived from allylamine, allyl-(3-amino-2-hydroxy-l-propyl) ether or oligo-or polyethylene glykol moieties of the formulae -CH=CH-CH₂-NH₂, -CH=CH-CH₂-O-CH₂-CH(OH)-CH₂-NH₂, and -O-(CH₂)ₙ-X-(CH₂)ₚ-NH₂ wherein X is -CH(-O(CH₂)ᵣ)ₛ)-, n is 1, p is zero, r is 2 and s is 1 to 20, preferably 1 to 3, respectively.

Preferably, the respective nucleoside trisphosphates have the following formula (I), 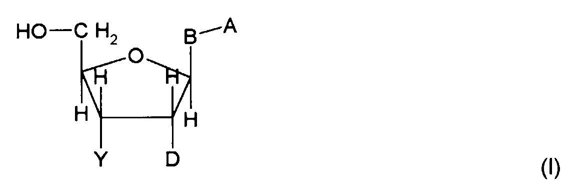 wherein Y is ―O-P[(=O)(OH]-O-P[(=O)(OH]-O-P[(=O)(OH]-OH;
A is -CH=CH-CH₂-NH₂, -CH=CH-CH₂-O-CH₂-CH(OH)-CH₂-NH₂, and -O-(CH₂)ₙ-X-(CH₂)ₚ-NH₂ wherein X is -CH(-O(CH₂)ᵣ) ₛ)-, n is 1, p is zero, r is 2 and s is 1 to 20, preferably 1 to 3, respectively; preferably A is ―CH=CH-CH₂-NH₂;
- B: is uracil;
- A: being bound to B in the 5-position of uracil; and
- D: is hydroxy or hydrogen.

These nucleoside trisphosphates, when built into the modified nucleic acids obtainable according to embodiment (A) of the invention, yield moieties of the formula 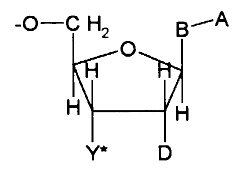 wherein Y^{*} is -O-P[(=O)(OH]-O- and the other symbols are defined as under formula (I).

A modified nucleic acid is a nucleic acid that comprises one or more (identical or, in the case more than one modified nucleoside is part of the molecule, different) modified nucleosides corresponding to a modified nucleoside trisphosphate mentioned above, with the proviso that at least one of the modified nucleosides is not terminally (3'-or 5'-terminally) bound to the rest of the molecule (strand). Such a modified nucleic acid is obtainable as described under embodiment (A).

The manufacture of modified nucleoside trisphosphates according to the invention is known in the art; for example, International Patent Application WO 89/12642, European Patent Application EP 0 063 879, and European Patent Application EP 0 192 297 each describe the methods for the synthesis of such or related compounds and are incorporated herein by reference.

Especially preferred are 5-allylamino UTP (AA-UTP) or 5-allylamino-deoxy UTP (AA-dUTP).

In embodiment (A) according to the invention, the modified nucleoside trisphosphate substrates are used prefereably in presence also of the corresponding unmodified nucleoside trisphosphate in order to result in the preferred modified nucleic acids with random distribution of modified nucleosides. For example, the may be used in a molar relation of unmodified:modified = 0.01-100 :1, preferably of 0.1 to 10:1, most preferably 1-2:1.

By way of the polymerase reaction, the step of multiplying or translating a nucleic acid double strand carrying modified nucleosides corresponding to the nucleoside trisphosphates mentioned above capable of covalent binding is produced (if e.g. one primer is used, only one of the strands in a modified DNA is modified, if two primers are used, it is possible to obtain DNA with two modified strands), or, in the case of RNA synthesis, a single strand (possibly with internal base pairing, e.g. in the case of loops or the like) capable of coupling, e.g. to a surface on a support, is produced.

The polymerase reactions are well-known in the art; the modified nucleoside trisphosphates mentioned above, especially the pyrimidine nucleoside triphosphates containing an allylamine moiety at the C-5 atom of the pyrimidine ring, especially AA-dUTP or AA-UTP, are good to excellent substrates for a wide variety of purified nucleic acid polymerases of both prokaryotic and eukaryotic origin. These include DNA polymerase I or E. coli, bacteriophage T4 DNA polymerase, DNA polymerases α and β from murine (A-9) and human (HeLa) cells, and the DNA polymerase of Herpes simplex virus. Confirming data are obtained with E. coli DNA polymerase I using either the nick-translation condition of Rigby, et al., J. Mol. Biol. 113, 237, 1977) or the gap-filling reaction described by Bourguignon et al., J. Virol. 20, 290, 1976). AA-dUTP can also be shown to function as a polymerase substrate both in CHO cells, permeabilized by treatment with lysolecithin according to the method of Miller, et al., Exp. Cell Res. 120, 421,1979) and in a nuclear replication system prepared from Herpes simplex infected BHK cells. For RNA manufacture, RNA polymerases of E. coli and bacteriophage T7, HeLa cell RNA polymerase III, calf thymus RNA polymerase II and mouse cell RNA polymerase II (eukaryotic RNA polymerases) can be used, or preferably RNA probes can be prepared enzymatically from DNA templates using E. coli or T7 RNA polymerases or by 3' end-labeling methods using RNA ligase with compounds such as AA-p(UTP)p. The AA- (= allylamine) and NAGE- (= allyl-(3-amino-2-hydroxy-1-propyl) ether) derivatives of UTP are especially well-suited substrates for the eukaryotic RNA polymerases mentioned above.

Using PCR methods, it is also possible to multiply an available nucleic acid.

It is also possible to start by first leaving away a modified nucleic acid and adding that only after an interruption or at a later time point during the polymerisation reaction, or to start first in the presence of a modified nucleotide trisphosphate and only after an interruption of or at a later time point during the reaction remove the modified nucleotide trisphosphate. It is also possible to use one or more modified nucleotide trisphosphates at the same time or at different times during the reaction. All these modifications also form part of the invention.

In the reaction to the modified nucleic acid, either only one modified nucleoside trisphosphate is used (leading to a nucleic acid with one type of modified nucleosides), or two or more different nucleic acids (e.g. one modified to carry a spacer and a thiol couling group, the other carrying a spacer and an amino coupling group) may be used (leading to a nucleic acid with two or more types of modified nucleosides).

A nucleic acid of interest for modification may be one from a sample of microbial (e.g. viral, bacterial (e.g. from Salmonella typhii) or fungal) or animal or plant origin, or the like, or a synthetic nucleic acid. However, non-synthetic nucleic acids are preferred, especially relatively large molecules with more than 100 nucleosides per strand.

A support according to the invention that can carry a covalently bonded modified nucleic acid obtainable by the method may be a solid support or a membrane, to the surface of which a modified nucleic acid is bonded.

A support is preferably a microbead, controlled pore-size glass, a chromatography matrix or especially a chip.

As chromatography matrix, any porous carrier material useful in chromatography is appropriate; preferred are materials that carry hydroxy or amino groups, e.g. aliphatic hydroxy or amino groups, such as polysaccharides, e.g. cross-linked dextranes or glucomannanes, hydroxy or amino group comprising poly(meth)acrylic acid derivatives and DIOL-modified or amino-modified silica gels. The pore size of the porous carrier material is preferably larger than would be appropriate for the specific separation problem to be solved. For example, DIOL- or amino-modified silica gels with pore sizes of 500 to 4000 Angstrom are used as preferred carrier materials.

Chips (also called "analysis chip", "array" or "grid") can be of substantially plain shape, or they can also have spheroid shape, or they can have the shape e.g. of microtiter plates (with several moulds/reaction chambers in array form).

The modified nucleic acids according to the invention can be brought to the surface of the chips by well-known methods, e.g. by photolithographic techniques or by spotting employing various contact or contact-free methods.

In one embodiment, the chip consists of one or more inorganic substances, such as glass, quartz glass, silica or the like, or of organic substances (for example polymers) or is a hybrid comprising different substances or layers of different substances (plastic coated glass, e.g. silane coated silica where the silane carries groups that allow for the covalent binding according to the invention). Usually, the support matrix is subsequently activated and/or a layer useful as coupling matrix is added. Thus, silanized surfaces, plasma-treated plastics or radiation- or radical-crafted polymers are possible. Other supports may be polystyrene, magnetic beads of the like.

The carrier for chips is preferably a plastic polymer accessible by injection moulding. As support material especially polycarbonate derivatives are suitable. The covalent coupling of the nucleic acid according to the invention preferably takes place on semifluid, gel-like polymer surfaces at the region of the samples (e.g. polymer brush). Semifluid polymer surfaces can be placed directly at the surface of the analysis chip and may be described as nano- to microstructures of surfaces, i.a. as polymer brushes. For an example of such chips see WO 99/57310 which is incorporated by reference. See also WO 99/36571 and
WO 99/57310, which are incorporated herewith by reference.

Immobilisation of the modified nucleic acid can be single of multiple. The bond between the nucleic acid and the support can be of any one or more of the types 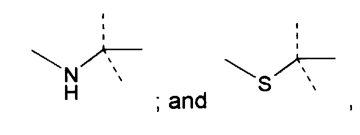 or others.

Preferred is the coupling via an amino group or a thiol group at the modified nucleoside(s).

The coupling takes place preferably to epoxy- or activated ester-functionalised surfaces, or carboxy-functionalised surfaces that are activated in situ e.g. by reaction with a coupling reagent useful in peptide chemistry; e.g. epoxy- or activated ester-functionalised or carboxy-functionalised polymer brushes. By using amino functions as coupling group of the modified nucleic acid, the product is sufficiently stable to allow its use in the printing process for DNA microarrays, e.g. on chips. The reaction conditions are in principle known in the art; for example, in the case of epoxy-activated surfaces, the reaction takes place in aqueous solvents (possibly in the presence of other solvents that are miscible with water, e.g. alcohol, such as ethanol or methanol, formamides, such as dimethylformamide, dioxane or the like), if desired in the presence of buffering substances, such as sodium phosphate or other buffering materials, at preferred temperatures in the range from 0 to 100 °C, preferably from 20 to 30 °C, e.g. at room temperature. It is also possible to use azide or diene group modifies surfaces (appropriate for Diels-Alder couplings with diene or azide comprising modified nucleosides, respectively).

Activated ester-functionalised surfaces are those obtainable by formation of an "active ester", e.g. an amino- or amido ester, such as a 1-hydroxy-benzotriazole (HOBT), succinimide or N-hydroxysuccinimide ester, or an aryl ester, such as a penta-fluorophenyl, 4-nitrophenyl or 2,4,5-trichlorophenyl ester (obtainable by treatment of the respective acid with a phenyl with the appropriate substituents, such as 4-nitrophenol or 2,4,5-trichlorophenol, and the like).

Activation of carboxy-functionalized surfaces in situ can be effected (i) directly with a carbodiimide, e.g. dicyclohexylcarbodiimide (DCC), N-ethyl-N'-(3-dimethylaminopropyl)-carbodiimide, N,N'-diethylcarbodiimide or N,N'-diisopropylcarbodiimide (DICD); with a carbonyl compound such as carbonyldiimidazole; with an 1,2-oxazolium compound, such as 2-ethyl-5-phenyl-1,2-oxazolium-3'-sulfonate and 2-tert-butyl-5-methylisoxazolium perchlorate; with an acylamino compound such as 2-ethoxy-1-ethoxycarbonyl-1,2-dihydroquinoline; with N-[(dimethylamino)-1H-1,2,3-triazolo[4,5-b]pyridin-1-ylmethylene]-N-methylmethanami-nium hexafluorophosphate N-oxide (HATU) ; with an uronium compound such as 2-(1H-benzotriazol-1-yl)-1,1,3,3-tetramethyluronium tetrafluoroborate (HBTU) or 2-(pyridon-1-yl)-1,1,3,3-tetramethyluronium tetrafluoroborate = O-(1,2-dihydro-2-oxo-1-pyridyl)-N,N,N',N'-tetramethyluronium-tetrafluoroborate (TPTU); or a phosphonium compound such as benzotriazol-1-yl-oxy-tris(dimethylamino)-phosphonium hexafluorophosphate (BOP) or benzotriazol-1-yl-oxy-pyrrolidino-phosphonium hexafluorophosphate (PyBOP); or

(ii) via formation of a symmetric anhydride (obtainable, for example, by condensation of the corresponding acid in the presence of a carbodiimide or 1-diethylaminopropyne; symmetric anhydrides method), or an asymmetric anhydride, such as the respective carbonic acid bromide, chloride or fluoride (obtainable, for example, by treatment of the respective carbonic acid with thionyl-, phosphopenta- or oxalyl-fluoride, -chloride or -bromide; acid halide (e.g. chloride) method).

Useful acid binding agents that can be employed in the condensation reactions of activated ester-functionalised surfaces or carboxy-functionalised surfaces are, for example, alkaline metal carbonates or bicarbonates, such as sodium or potassium carbonate or bicarbonate (if appropriate, together with a sulfate), or organic bases such as sterically hindered organic nitrogen bases, for example tri-lower alkylamines, such as N,N-diisopropyl-N-ethylamine, pyridine, N-lower alkylmorpholine, such as N-methylmorpholine, or N-methyl-pyrrolidin-2-one (the latter can also be used as solvent), which can be used alone or in any appropriate combination.

The reaction takes place in aqueous solvents (possibly in the presence of other solvents that are miscible with water, e.g. alcohol, such as ethanol or methanol, formamides, such as dimethylformamide, dioxane or the like), at preferred temperatures in the range from 0 to 100 °C, preferably from 20 to 30 °C, e.g. at room temperature.

The reaction to obtain a matrix that is gel-like and that is obtainable by cross-linking of a modified nucleid acid according to the invention may be made under conditions and employing reagents that are well-known in the art.

As cross-linkers, homo-bi- (or in a broader embodiment also tri-) functional cross-linking reagents are preferred, especially in the case where the modified nucleic acid to be cross-linked has amino coupling groups at the modified nucleosides. Such cross-linkers are, for example, diisothiocyanates, especially 1,3-diisothiocyano-benzene, bis-succinimidyl derivatives, especially disuccinimidyl glutarate, dithiobis(succinimidoylpropionate), disuccinimidoyl suberate, disuccinimodoyl tartrate, ethylen glycol-bis(succinimidylsuccinate) or bis(2-(succinimidyloxycarbonyloxy)ethyl)sulfone, methyl imidates, such as dimethyl pimelidate, dimethyl-3,3'-dithiobispropionimidate, dimethyl-adipimidate or dimethylsuberimidate (as hydrochloride salts), sulfosuccinimidoyl derivatives, such as 3,3'-dithiobis(sulfosuccinimidyl proprionate), disulfosuccinimidyl tartrate, ethylene glycol-bis(sulfosuccinimidylsuccinate), bis-(sulfosuccinimidoyl)-suberate or bis(2-(sulfosuccinimidooxycarbonyloxy)ethyl)sulfone, bis-carbodiimides, such as 1-ethyl-3-(3-dimethylaminopropyl)carbodiimide hydrochloride, or 1,5-difluoro-2,4-dinitro-benzene.

In a broader aspect of the invention, the following cross-linkers may also be used:

For cross-linking of sulfhydryl coupling group comprising reagents, especially sulfhydryl-reactive homobifunctional cross-linkers can be used, e.g. bis-maleimides, such as dithio-bismaleidoethane, bis-vinyl sulfones, such as 1,6-hexane-bis-vinylsulfone, 1,4-bis-maleimidobutane, 1,4-bis-maleimidyl-2,3-dihydroxybutane, bis-maleimidohexane, bis-maleimido-ethane, 1,8-bis-maleimidotriethylenglycol or 1,11-bis-maleimidotetraethylenglycol.

Of course, it is also possible to use hetero-bifunctional cross-linking reagents, e.g. reagents that comprise one group that reacts with amino, hydroxy or mercapto, and another group that is a disulfide that may subsequently be reduced to yield free mercapto groups (e.g. with dithio-threitol or the like) that may then be reacted with each other to result in a cross-linked matrix by mild oxidation.

This is especially preferred where the modified nucleosides contain mercapto and (e.g. at different sites) amino, mercapto or hydroxy groups that can be cross-linked.

Other possible hetero-bifunctional cross-linkers are, e.g., cross-linkers carrying one amino-reactive group and a photoactivatable group. Here, first chemical reaction of the amino-reactive group leads to modification of amino-coupling group containing modified nucleosides, and subsequently photoreaction can be used for the cross-linking. Examples of such cross linkers are N-hydroxysuccinimidoyl-4-azido-salicylic acid, N-succinmidoyl-(4-azidophenyl)-1,3-dithiopropoinate, sulfosuccinimidoyl-2-(7-azido-4-methylcoumarin-3-acetamido)ethyl-1,3'-dithiopropionate, sulfosuccinimidoyl-2-(m-azido-o-nitrobenzamido)ethyl-1,3'-dithiopropionate, N-succinimidyl-6-(4'-azido-2'-nitro-phenylamino)hexanoate, sulfosuccinimidyl-2-(p-azidosalicylamido)ethyl-1,3'-dithiopropionate, N-hydroxysulfosuccinimidoyl-4-azido-benzoate, sulfosuccinimidoyl(4-azidosalicylamino)hexanoate, sulfosuccinimidyl(4-azidophenylthio)propionate, sulfosuccinimidoyl-6-(4'-azido-2'-nitrophenylamino)hexanoate, n-(4-(p-azidosalicylamido)butyryl)-3'-(2'-pyridyldithio)propionamide, or bis-(beta-(-azidosalicylamido)ethyl)disulfide.

Further possible hetero-bifuncational cross-linkers are, for example, reagents that comprise one amino- and one sulfide-reactive group or two amino-reactive groups, respectively, for example Succinimidoyl-maleinimid derivatives, such as succinimidoyl-butylphenyl-maleinimide, N-ε-maleimidocaproic acid, or N-(ε-maleimidocaproic acid) hydrazide, N-(ε-maleimidocaproiyloxy) succinimide ester, N-(γ-maleimidobutyryloxy)succinimide ester, N-κ―maleimidoundecanoic acid or its N-hydrazide, succinimidyl-4-(N-maleimidomethyl)cyclohexane-1-carboxy-(6-amidocaproate), succinimidyl-6-(3-(2-pyridyldithio)propionamido)hexanoate, m-maleimidobenzoyl-N-hydroxysuccinimide, succinimidoyl-3-(bromoacetamido)propionate, N-(ε-maleimidocaproyloxy)sulfosuccinimide ester, N-(γ-maleimidobutyryloxy)sulfosuccinimide ester, sulfosuccinimidoyl-4(ρ-maleimidophenyl)butyrate, sulfosuccinimidyl-6-(α-methyl-α-(2-pyridyldithio)toluamideo)hexanoate, N-(α-maleimidoacetoxy)succinimidate ester or N-(betamaleimidopropyloxy)succinimde ester; or in the case of two amino-reactive groups methyl-N-succinimidoyl adipate.

An example for sulfhydryl- and hydroxyl-reactive heterobifunctional cross-linkers is N-(p-maleimidophenyl)isocyanate.

It is possible to manufacture cross-linked nucleic acid, e.g. polynucleotide, gels that can be used as matrix for affinity chromatography. For example, in a preferred embodiment of the invention, AA-dUTP modified poly dT/dU (appropriate e.g. for separating eukaryotic poly-A-comprising mRNA from other RNA) can be produced by polymerisation of an dUTP/AA-dUTP mixture using terminal deoxynucleotide transferase and may then be cross-linked employing homo-bifunctional cross-linkers, such as 1,3-diisothiocyanobenzene, in order to obtain a cross-linked gel.

A kit comprising a modified support or a matrix according to the present invention consists preferably of the modified support, e.g. a chip, and, if required, reagents that are necessary for its use, e.g. buffers (either in dissolved form or in dry form where water has to be added), one or more antibodies, e.g. primary or secondary antibodies to detect DNA or certain DNA-binding proteins, labelling reagents, such a fluorescence labels or biotin or labelled proteins, e.g. antibodies, avidin, streptavidin or the like, detection devices, such as UV detectors or fluorescence detectors, and/or any other reagent or device useful for the analysis or purification of the desired sample.

The kit can be used for analysis or purification purposes, e.g. for the purpose of RNA expression profiling or mutation profiling (e.g. in order to enrich RNA by adsorption or to remove RNA (e.g. ribosomal RNA by binding to complementary modified nucleic acids that are cross-linked in the matrix) from the liquid phase, leaving only the non-binding RNA (e.g. the non-ribosomal RNA = mRNA) in the liquid phase for further analysis) or nucleic acid binding (e.g. regulatory) proteins, e.g. derived from food, microorganisms (bacteria, yeasts, algae, viruses), degenerate, e.g. tumor, cells, plants, fungi, animals, for enrichment of certain molecules, e.g. by affinity chromatography, e.g. nucleic acid binding proteins, nucleic acids capable of hybridisation; and the like.

As an example, unknown DNA samples can first be amplified, then, using the method of the invention, be subjected to introduction of modified nucleotides (e.g. by polymerase reaction in the presence of modified nucleotides) that allow for covalent binding to a support, and then be detected using known probes, e.g. labelled DNA, for example by way of hybridisation. As the DNA maintains its integrity, it is also possible to detect DNA binding proteins, such as regulatory proteins, and find out to which nucleic acid e.g. on an array they bind. This might, e.g., be done by antibodies specific to such a nucleic acid binding protein (if required involving secondary and/or tertiary antibodies, or by labelling such protein directly).

Wherever a support or matrix or a kit comprising such support or matrix carrying a covalently bonded nucleic acid (obtainable according to embodiment (A) mentioned above) is used for analysis or purification according to the present invention, the following conditions are useful and thus preferred for hybridisation:

For the hybridisation conditions, the setting of conditions that allow for determining if strictly complementary sequences or sequences with a higher or lower degree of complementarity sequences or sequences with a higher or lower degree of homology are present is well known to the person skilled in the art. Appropriate conditions or protocols can be found, e.g., in standard books, e.g. in Sambrook et al., "Molecular Cloning, A Laboratory Manual", CSH Press, Cold Spring Harbor, 1989, or in Hames and Higgins, "Nucleic acid hybridisation, a practical approach", IRL Press, Oxford (1985). This, the detection of only specifically hybridising sequences will usually require stringent hybridisation and washing conditions, such as 0.1xSSC, 0.1 % SDS at 65 °C. Non-stringent hybridisation conditions for the detection of homologous and not exactly complementary sequences may be set at 6xSSC, 1%SDS at 65 °C. As is well known, the length of the probe and the composition of the nucleic acid (e.g. prevalence of A-T or G-C base pairs) constitute further parameters that influence the hybridisation conditions.

In order to disrupt, if a double strand DNA molecule has been produced by the method according to embodiment (A) of the invention, the base parings for obtaining single strands, it is possible to use base denaturation or especially heat denaturation, but also strand displacement reactions by polymerase, chemical or enzymatic degradation of one strand, or any appropriate combination thereof.

The detection of hybrids can be carried out by a variety of known methods, usually depending on the labeling that is employed. For example, if an amplification step is involved (see eg.g. WO 99/36571) that uses fluorochrome-labeled oligonucleotides, the detection may be by visual means, for example with a microscope equipped appropriately. Other options include capillary and gel electrophoresis, HPLC, mass spectroscopy, nucleic acid hybridisation and nucleic acid sequencing. One preferred embodiment also applicable to other embodiments involves the detection by using an anti-double-stranded DNA antibody. Said antibody may be detectably labeled, or it may be detected using, e.g., a secondary antibody that is detectably labeled. Another preferred embodiment employed for detection is hybridisation with a detectably labeled probe.

One of the characteristic features of supports carrying modified nucleotides according to the invention is that, even if the covalently bonded nucleic acid is a double stranded DNA, it can still be used for the detection of hybridisation with nucleic acids from a sample, e.g. double-stranded DNA, single-stranded DNA or RNA. It is then necessary

In the case where only one of the strands of a DNA to be bonded is modified, e.g. by use of a single primer only, it is also possible to first denature the nucleic acid, thus providing single strands, e.g. by any one of the methods just described, and then coupling only this one strand to the support as described under embodiment (D) above.

This method is one of the preferred embodiments according to the invention. Another preferred embodiment is DNA bonded to the support via two modified nucleic acid strands, obtainable e.g. as described above by use of two primers in the polymerase reaction leading to the modified DNA.

Labelled nucleic acid probes that can be used in hybridisation reactions according to the invention (e.g. in order to analyse a modified nucleic acid according to the invention by using known nucleic acid sequences) may be prepared by a number of methods, e.g. by random priming (employing the Kleenow enzyme = large subtilisin fragment of E-coli DNS polymerase holoenzyme; the template is dsDNA, the labeling is made at a hexamer of dNTP; resuits in a mixture of probes of varying lengths, where all partial sequences are specific); by nick-translation (using pancreatic Dnase I plus E. coli DNA polymerase holoenzyme; the template is dsDNA, the labeling takes place at dNTP; it is necessary to use exactly determined enzyme concentration; less than 100 % labeling yield); PCR amplification (DNA polymerase, e.g. Taq-, Tth-, Pwo, Pfu-polymerase); applicable to ssDNA or dsDNA; labeling at the primer or dNTP; variable affinity of the polymerase to modified nucleotides); reverse transcription (using reverse transcriptase of viral origin (AMV or MoMV-RT); applicable to ssRNA; labeling at the dNTP; the probe amount is depending on the amount of RNA used); terminal transferase reaction (using terminal transferase; applicable to ssDNA; labeling at the dNTP; length of the resulting chains depends on reaction conditions); or run-off transcription (using RNA-polymerase, codes by bacteriophages (SP6, T3, T7); applicable to dsDNA, labeling at dNTP; results in a fusion construct; low rates of incorporation). [dsDNA = double-stranded DNA, ssDNA = single stranded DNA, ssRNA = single stranded RNA; dNTP = deoxynucleotide trisphosphate.]

A kit for introducing modified nucleosides according to the invention and, if desired, then covalently binding such a nucleic acid to a support may comprise any one or more of the reagents and substrates defined above for embodiments (A) (especially an appropriate polymerase and one or more modified nucleoside trisphosphates) and (D) (especially using chips as support).

### Examples:

The following examples serve to illustrate the invention, but are not thought to limit its scope in any way.

### Example 1: Functionalisation of PCR amplificates by incorporation if allyamino deoxyuracil trisphosphate (AA-dUTP) by way of amplification of a DNA using thermostable DNA polymerases:

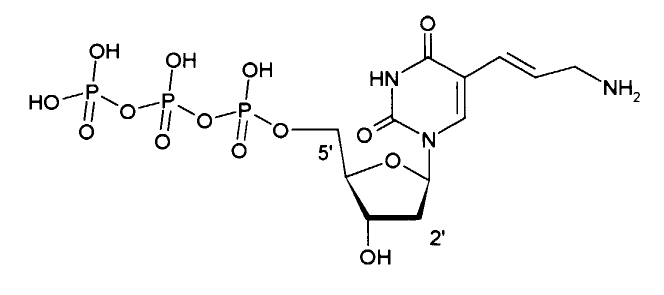

100 pg of a purified PCR product (a genomic DNA obtained from Salmonella typhii, comprising 428 bp (base pairs)) are amplified using polymerase chain reaction using taq-polymerase from Quiagen in a buffer consisting of 2 mM dATP, dCTP, dGTP, 1.5 mM dUTP, 1 mM AA-dUTP (obtainable from Sigma) and 4 mM MgCl₂ using 40 cycles in a thermocycler (PE9700). After the initial denaturation at 94 °C for 7 minutes the template is amplified in 40 cycles (94 °C: 30 sec, 60 °C: 30 sec, 72 °C: 60 sec). After a final elongation time of 10 min at 72 °C the DNA is separated on an agarose gel and obtained in purified form by elution. The degree of incorporation is determined by reaction with fluorescein isothiocyanate and subsequent photometric determination of the FITC and DNA concentration. The resulting modified DNA is thus determined to carry ca. one modified base per 100 bases. The suitability of the product for coupling to a solid or semi-solid surface is determined by reaction with glycidoxy-functionalised polymer brushes (oxirane-carrying methacrylate derivative, obtainable from Fluka) 0.1 µM AA-dUTP-labelled PCR product is dissolved in a 100 mM sodium phosphate solution and pipetted in volumes of each 200 nl onto the support. Uncoupled product is washed out with sodium phosphate buffer. As control, a non-modified DNA of the same concentration is employed which does binds significantly less under the above-mentioned conditions, and an aminoterminated oligonucleotide with a fluorescent marker that binds. Subsequently, the functionality of the bond is evidenced by hybridisation with fluorescence-labeled PCR products (Cy5 = carbocyanin modified DNA).

### Example 2: Manufacture of a modified RNA from PCR amplificates by incorporation if allyamino uracil trisphosphate (AA-UTP):

Using T7 polymerase, a transcript is obtained from PCR amplificates of the same genomic DNA obtained from Salmonella typhii, comprising 428 bp, used in example 1, the forward primer of which in addition contains a 22 nucleoside long T7 polymerase promoter (e.g. sequence TAATACGACTCACTATAGGGAG). In a 20 µl incubation, 2 µl of template (= 1 µg/ml) are incubated with 2 µl T7-reaction buffer (Roche Diagnostics), 4 µl NTP mix (2.5 mM ATP. CTP, GTP, 1mM UTP, 1.5 mM AA-UTP, 2 µl T7 polymerase (190 ng/µl) for 2 h at 37 °C and precipitated subsequently with LiCl/ethanol. The RNA after this reaction contains the amino groups that are detected as in example 1.

### Example 3: Manufacture of an affinity matrix:

Bacterial cRNA of rRNA (e.g. obtainable from Salmonella typhii) and tRNA genes is cross-linked with 1,3-diisothiocyanobenzene.

First, the cRNA is obtained from cloned rRNA and tRNA genes using the T7 promoter also used in example 2, as well as 1mM UTP and 1.5 mM AA-UTP, under reaction conditions as described in example 2.

100 µl of the resulting sample (1 µg/µl, determined photometrically) of single-stranded cRNA are incubated in 100 mM sodium phosphate buffer, pH 8.0, with 10 mM 1,3-diisothiocyanobenzene and shaken at 37 °C for 1 h. After that, the resulting material is precipitated with a 2.5-fold excess of ethanol. The precipitated gel is then transferred with a pipette to a glass frit of 5 µM pore size, thus yielding a miniature "column".

The frit "column" is then used for binding rRNA and tRNA from bacterial samples, the eluate merely containing mRNA that can then be used for further purposes, .

## Claims

1. A method or process for the manufacture of a modified nucleic acid, comprising the steps of multiplying (or translating) an available nucleic acid using a DNA or RNA polymerase, where, during the process, either one modified nucleoside trisphosphate or more than one non-identical modified nucleoside trisphosphates are used as substrate in order to obtain a nucleic acid wherein at least one of the modified nucleosides is present and at least one modified nucleoside is bonded non-terminally.

2. The method according to claim 1 wherein both the modified nucleic acid and its unmodified analogue are present during the polymerase reaction, preferably in a molar relation of unmodified:modified = 0.01-100:1, preferably of 0.1 to 10:1, most preferably 1-2:1.

3. The method according to claim 1 wherein the modified nucleoside trisphosphate has the following formula (I), 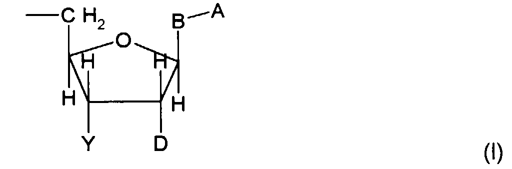 wherein Y is ―O-P[(=O)(OH]-O-P[(=O)(OH]-O-P[(=O)(OH]-OH;
A is a spacer carrying a coupling group;
B is a base; and
D is hydroxy or hydrogen.

4. The method according to claim 3 wherein in the respective nucleoside trisphosphate of the following formula (I)
Y is ―O-P[(=O)(OH]-O-P[(=O)(OH]-O-P[(=O)(OH]-OH;
A is ―CH=CH-CH₂-NH₂;
B is uracil;
A being bound to B in the 5-position of uracil; and
D is hydroxy or hydrogen.

5. A method according to any one of claims 1 to 4, wherein a DNA is manufactured wherein only one of the strands carries modified nucleosides.

6. A modified nucleic acid obtainable according to the method or process described in any one of claim 1 to 5, especially a double-stranded DNA that comprises modified nucleosides either in one (claim 5) or in both strands (claims 1 to 4), or an RNA that carries such modified nucleosides, with the proviso, that at least one of the binding modified nucleosides is not terminally bound to the respective modified DNA or RNA, or preferably that no modified nucleoside is present terminally in the respective modified nucleic acid strand(s).

7. A support carrying a covalently bonded modified nucleic acid, especially a DNA or RNA, where the nucleic acid is bonded via one or more modified nucleosides to the surface of said support, with the proviso, that at least one of the binding modified nucleosides is not terminally bound to the respective modified DNA or RNA, or preferably that no modified nucleoside is present terminally in the respective modified nucleic acid strand(s).

8. A support according to claim 7 which is a chip.

9. A method of manufacturing a support carrying a covalently bonded modified nucleic acid, especially a DNA or RNA, where the nucleic acid is bonded via one or more modified nucleosides to the surface of said support, with the proviso that at least one of the binding modified nucleosides is not terminally bound to the respective modified DNA or RNA, or preferably that no modified nucleoside is present terminally in the respective modified nucleic acid strand, comprising the step of reacting a modified nucleic acid obtainable according to any one of claims 1 to 5 with a support.

10. A matrix obtainable by cross-linking a nucleic acid obtainable according to any one of claims 1 to 5 with a cross-linker.

11. A matrix according to claim 10 where the cross-linker is a homo-bifunctional cross-linker selected from the group consisting of diisothiocyanates, especially 1,3-diisothiocyano-benzene, bis-succinimidyl derivatives, especially disuccinimidyl glutarate, dithiobis(succinimidoylpropionate), disuccinimidoyl suberate, disuccinimodoyl tartrate, ethylen glycol-bis(succinimidylsuccinate) or bis(2-(succinimidyloxycarbonyloxy)ethyl)sulfone, methyl imidates, such as dimethyl pimelidate, dimethyl-3,3'-dithiobispropionimidate, dimethyl-adipimidate or dimethylsuberimidate (as hydrochloride salts), sulfosuccinimidoyl derivatives, such as 3,3'-dithiobis(sulfosuccinimidyl proprionate), disulfosuccinimidyl tartrate, ethylene glycol-bis(sulfosuccinimidylsuccinate), bis-(sulfosuccinimidoyl)-suberate or bis(2-(sulfosuccinimidooxycarbonyloxy)ethyl)sulfone, bis-carbodiimides, such as 1-ethyl-3-(3-dimethylaminopropyl)carbodiimide hydrochloride, or 1,5-difluoro-2,4-dinitro-benzene; and where the nucleic acid is one obtainable according to claim 4.

12. A method for the manufacture of a matrix according to any one of claims 10 and 11, wherein a nucleic acid obtainable according to any one of claims 1 to 5 is cross-linked in the presence of a cross-linker according to any one of claims 10 and 11.

13. A kit comprising a support carrying a covalently bonded modified nucleic acid, especially a DNA or RNA, or a matrix obtainable by cross-linking said nucleic acid; where the nucleic acid is bonded via one or more modified nucleosides (which may be of one or more types) to the surface of said support or matrix, with the proviso that at least one of the binding modified nucleosides is not terminally bonded to the respective modified DNA or RNA, or preferably that no modified nucleoside is present terminally in the respective modified nucleic acid strand(s).

14. The use of a kit according to claim 13 for the analysis or purification of nucleic acid or protein samples.

15. A kit for introducing modified nucleosides into a nucleic acid according to the method described in any one of claims 1 to 5 and, if desired, then covalently binding such a nucleic acid to a support.

16. A process for the manufacture of a modified nucleic acid, especially a DNA or RNA, especially carrying a coupling group capable of binding covalently to a support or to other molecules, comprising the steps of multiplying (or translating) an available nucleic acid, especially a DNA or RNA, using a DNA or RNA polymerase, where, during the process, either one modified nucleoside trisphosphate or more than one non-identical modified nucleoside trisphosphates are used as substrate in order to obtain a nucleic acid wherein at least one of the modified nucleosides is present and at least one modified nucleoside is bonded non-terminally; and then manufacturing a support carrying a covalently bonded modified nucleic acid, especially a DNA or RNA, where the nucleic acid is bonded via one or more modified nucleosides (of one or more types) to the surface of said support, with the proviso that at least one of the binding modified nucleosides is not terminally bound to the respective modified DNA or RNA, or preferably that no modified nucleoside is present terminally in the respective modified nucleic acid strand, by reacting said modified nucleic acid with a support.
